# EUROPEAN PATENT SPECIFICATION

(11) **EP 2 508 662 B1**
(45) Date of publication and mention of the grant of the patent: **10.09.2014**
(21) Application number: 10833109.1
(22) Date of filing: 17.11.2010
(51) Int. Cl.: D04H 1/48, A61F 13/15, A61F 13/49, A61F 13/53, D04H 1/40, D04H 1/42

(54) **METHOD FOR REDUCING THICKNESS OF MASS OF WATER-ABSORBING MATERIAL AND THIN MASS OF WATER-ABSORBING MATERIAL OBTAINED BY THE METHOD**
VERFAHREN ZUR REDUZIERUNG DER DICKE EINER MASSE AUS WASSERABSORBIERENDEM MATERIAL UND IN DIESEM VERFAHREN GEWONNENE DÜNNE MASSE AUS WASSERABSORBIERENDEM MATERIAL
PROCÉDÉ DE RÉDUCTION DE L'ÉPAISSEUR D'UNE MASSE DE MATÉRIAU HYDROPHILE ET MASSE FINE DE MATÉRIAU HYDROPHILE OBTENUE PAR LE PROCÉDÉ

(30) Priority: 30.11.2009 JP 2009272890
(43) Date of publication of application: 10.10.2012
(73) Proprietor: Unicharm Corporation, Ehime 799-0111 (JP)
(72) Inventor: GODA, Hiroki, Kanonji-shi Kagawa 769-1602 (JP); MIZUTANI, Satoshi, Kanonji-shi Kagawa 769-1602 (JP)
(74) Representative: Eke, Philippa Dianne
(86) International application number: PCT/JP2010/070439
(87) International publication number: WO 2011/065262

(56) References cited:
- DE-A1-102008 007 804
- JP-A- 54 123 293
- JP-A- 2004 238 785

## Description

### {Technical Field}

The present invention relates to methods for thinning an aggregate of water-absorbent materials adapted to be used as a water-absorbent core in a disposable bodily fluid absorbent article such as a disposable diaper, a menstruation napkin and a urine absorbent pad and also relates to thin aggregates of water-absorbent materials obtained using such methods.

### {Background}

It is known to use an aggregate of water-absorbent materials including fluff pulp fibers and the other hydrophilic fibers as a water-absorbent core in a bodily fluid absorbent disposable article and it is also known to use an aggregate of water-absorbent materials including hydrophilic fibers and superabsorbent polymer particles for this purpose. It is also known to compress the core in its thickness direction to obtain a thin type core before use in the bodily fluid absorbent disposable article, considering that the core including the hydrophilic fibers is apt to become bulky.

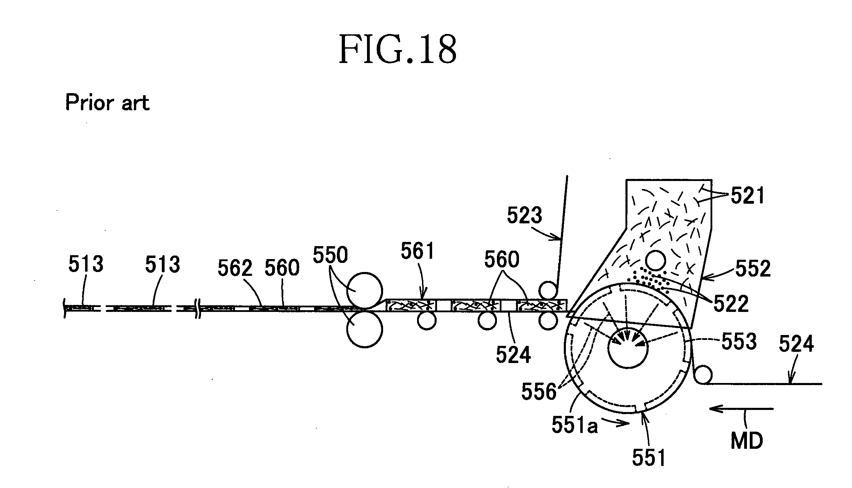

Fig. 18 of the accompanying drawings exemplarily illustrates a process of the prior art used to make water-absorbent cores. In the illustrated process of the prior art, a second web 524 formed of a continuous tissue paper is fed from the upstream side in a machine direction MD to a peripheral surface 551a of a suction roll 551. The peripheral surface 551a is subjected to a suction effect 556 directed towards a center of the suction roll 551. The second web 524 is transported in a feeding region 552 for water-absorbent materials and sucked into concave portions 553 formed in the peripheral surface 551a of the roll 551 under the suction effect 556. In the feeding region 552, fluff pulp fibers 521 and superabsorbent polymer particles 522 are accumulated within the respective concave portions 553 also under the suction effect 556 to form aggregates 560 of the water-absorbent materials 560. The aggregates 560 having left the feeding region 552 are sandwiched between a first web 523 formed of a continuous tissue paper fed from above and the second web 524 on which the aggregates 560 are placed to form first composite web 561. This first composite web 561 is transported in the machine direction MD and compressed by a pair of press rolls 550 into a second composite web 562 having a desired thickness. This thickness can be adjusted by changing a nip of these press rolls 550. The second composite web 562 having passed by the press rolls 550 is cut between each pair of the adjacent aggregates 560 to obtain individual water-absorbent cores 513.

The core for a disposable diaper according to the invention disclosed in US Patent No. 3,938,522 B (PTL 1) contains fluff pulp fibers wherein fluff pulp fibers are transported in the form of a web in the machine direction and once pressed by calendar rolls, then water sprayed and compressed again by calendar rolls.

The invention disclosed in JP 2512415 B2 (PTL 2) provides an absorbent structure in the form of an air-jet papermaking processed and dried mixture comprising hydrophilic fibers and discrete hydro gel particles of a water-insoluble crosslinked polymer. This absorbent structure has a density in a range of about 0.15 to about 1g/cm³, a moisture content less than about 10% by mass and a Gurley stiffness less than 2g and is flexible and substantially in a non-bonded state. For the method for making the absorbent structure disclosed in PTL 2, it is essential to compress the air-jet papermaking processed and dried mixture of the hydrophilic fibers and the water-insoluble hydro gel particles until the dried mixture has a density in a range of about 0.15 to about 1g/cm³.

### {Citation List}

### {Patent Literature}

{PTL 1} US 3,938,522 B
{PTL 2} JP 2512415 B2

### {Summary of Invention}

### {Technical Problem}

In an aggregate of water-absorbent materials used in the bodily fluid absorbent disposable article including hydrophilic fibers, such aggregate is inevitably apt to become undesirably thick. Considering this, the aggregate is preferably compressed to become as thin as possible to assure that this aggregate may not be bulky and may create a comfortable feeling to the wearer when the bodily fluid absorbent disposable article including this aggregate is put on the wearer's body. However, when the aggregate is compressed to a desired thickness by using the method of the prior art, in consideration of a recovery of a thickness after compression, it is necessary to compress the aggregate into a further smaller thickness than a thickness actually required for the finished aggregate. In the aggregate containing superabsorbent polymer particles, such surplus compression may collapse a proper shape of each of the super absorbent polymer particles and cause polymer components having a low crosslink density to be exposed. In consequence, the superabsorbent polymer particles having absorbed water may easily form a gel block. The aggregate formed with the gel block in this manner will prevent the superabsorbent polymer particles confined within this gel block from coming in contact with bodily fluids and thereby preventing the aggregate from fulfilling its function as the absorber. Consequentially, the amount of bodily fluids of the core including this aggregate may be noticeably decreased and/or the absorption rate for bodily fluids of the core may be noticeably deteriorated. In addition, excessive compression may locally develop an undesirable state for the aggregate in which the hydrophilic fibers come in close contact with each other and/or the hydrophilic fibers come in close contact with the superabsorbent polymer particles to form regions exhibiting extremely high stiffness. This may lead to the core which is uneven in its flexibility and water-absorbability.

An object of the present invention is to provide a method for thinning an aggregate of water-absorbent materials including hydrophilic fibers and superabsorbent polymer particles and to provide a sufficiently thin aggregate of water-absorbent materials obtained by this method.

### {Solution to Problem}

The present invention includes a first aspect relating to a method for thinning the aggregate of water-absorbent materials and a second aspect relating to a sufficiently thin aggregate of water-absorbent materials obtained by the method according to the first aspect.

According to the first aspect of the present invention, there is provided a method for thinning the aggregate of water-absorbent materials including hydrophilic fibers and superabsorbent polymer particles in a thickness direction of the aggregate.

In this method, the first aspect of the present invention includes a step of ejecting steam streams at a temperature corresponding to water's boiling point or higher against the aggregate while this aggregate is compressed in the thickness direction to thin the aggregate.

The aggregate is sandwiched and compressed by a pair of air-permeable supporting means opposed to each other in the thickness direction of the aggregate while the steam streams are ejected against the aggregate in the thickness direction through one of the pair of air-permeable supporting means.

According to one embodiment of the first aspect of the present invention, the steam streams are one of moist steam streams, saturated steam streams and dry steam streams.

According to another embodiment of the first aspect of the present invention, the steam streams are high pressure steam streams at a steam pressure in a range of 0.1 to 2.0MPa.

According to yet another embodiment of the first aspect of the present invention, the steam is sucked under vacuum suction effect after the steam streams has been ejected against the aggregate and has passed through the aggregate.

According to a further embodiment of the first aspect of the present invention, the steam in a range of 1.23kg/m² to 0.03kg/m² of surface area of the aggregate facing one of the pair of air-permeable supporting means is ejected while the pair of air-permeable supporting means run at a velocity in a range of 5 to 500m/min in one direction.

According to an alternative embodiment of the first aspect of the present invention, the pair of air-permeable supporting means respectively include segments adapted to sandwich the aggregate and to run in one of a horizontal direction, a vertical direction and a tilted direction between these horizontal and vertical directions.

According to another alternative embodiment of the first aspect of the present invention, each of the pair of air-permeable supporting means is an endless belt.

According to still another alternative embodiment of the first aspect of the present invention, the aggregate is previously thinned by mechanically compressing the aggregate in the thickness direction and thereafter the steam streams are ejected against the aggregate kept under compression.

According to yet another alternative embodiment of the first aspect of the present invention, the aggregate is mechanically compressed using at least a pair of pressure rolls for previously thinning the aggregate.

According to a further alternative embodiment of the first aspect of the present invention, at least one of a side facing one of the pair of air-permeable supporting means and a side facing the other of the pair of air-permeable supporting means of the aggregate is covered with one of an air-permeable sheet and an air-permeable and liquid-pervious sheet and thereafter the aggregate is sandwiched between the pair of air-permeable supporting means.

According to a varied embodiment of the first aspect of the present invention, after being subjected to ejection of the steam streams, one of a side of the aggregate facing the one of the pair of air-permeable supporting means and the other side of the aggregate facing the other of the pair of air-permeable supporting means is covered with one of an air-permeable sheet, an air-permeable and liquid-pervious sheet and a non-air-permeable sheet.

According to another varied embodiment of the first aspect of the present invention, in the step of separating the aggregate, after having been subjected to ejection of the steam streams, from the one of the pair of air-permeable supporting means and the other of the pair of air-permeable supporting means, the aggregate is subjected to the vacuum suction effect through any one of the pair of air-permeable supporting means.

According to still another varied embodiment of the first aspect of the present invention, the aggregate includes the hydrophilic fibers in a range of 98 to 10% by mass and the superabsorbent polymer particles in a range of 2 to 90% by mass.

According to yet another varied embodiment of the first aspect of the present invention, the hydrophilic fibers may be selected from a group including fluff pulp fibers, cotton fibers, rayon fibers, acetate fibers and thermoplastic synthetic fibers modified to become hydrophilic.

According to a further varied embodiment of the first aspect of the present invention, the superabsorbent polymer particles may be selected from particles of polyacrylic acid, polyacrylate, starch-acrylonitrile graft copolymer, polyvinyl alcohol, polyvinyl ether, polyacrylamide, carboxymethyl cellulose or natural polysaccharide.

According to a modified embodiment of the first aspect of the present invention, one of the air-permeable sheet and the air-permeable and liquid-pervious sheet is one of a tissue paper and a nonwoven fabric.

According to the second aspect of the present invention, provided is a sufficiently thin aggregate of water-absorbent materials made by the method according to the first aspect of the present invention.

### {Advantageous Effects of Invention}

In the method according to the present invention to thin the aggregate of water-absorbent material and the aggregate of water-absorbent material made sufficiently thin using this method, steam streams at a temperature corresponding to water's boiling point or higher are ejected against the aggregate of water-absorbent material while the aggregate of water-absorbent material is mechanically compressed. In this way, the aggregate of water-absorbent material as a whole in its thickness direction can be quickly brought into a heated and humidified state or a heated state and thereby the aggregate can be easily thinned. Specifically, the hydrophilic fibers in the aggregate can be easily deformed under the effect of steam streams ejected thereagainst without requiring a significantly high mechanical compression and the aggregate once deformed under the effect of steam is slow to restore its initial shape. The aggregate including such hydrophilic fibers can be also quickly thinned and is slow to restore its initial thickness. The aggregate may be compressed in this manner to prevent the superabsorbent polymer particles included in the aggregate from losing the shape thereof. In the course of the method according to the present invention to thin the aggregate of water-absorbent material, the superabsorbent polymer particles would not partially or wholly collapse even when the superabsorbent polymer particles absorb water and such an aggregate of water-absorbent material may be well resistant to formation of gel block. Thus the problems due to formation of gel block may be prevented from occurring.

### {Brief Description of Drawings}

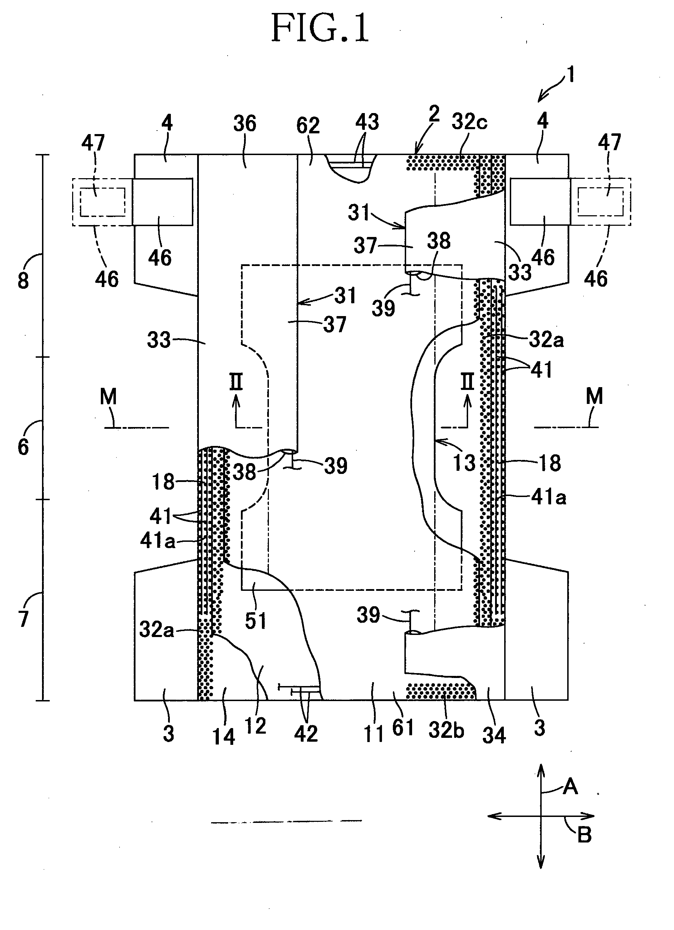

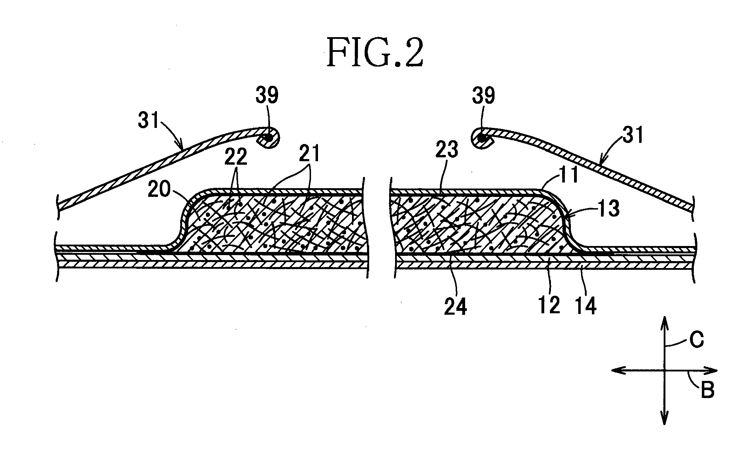

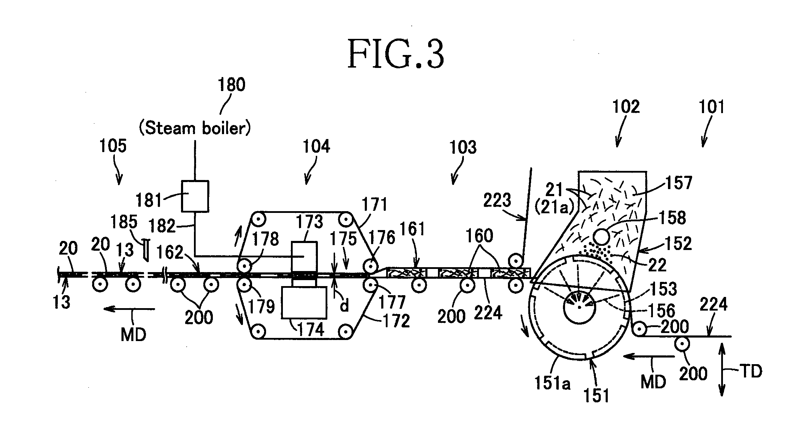

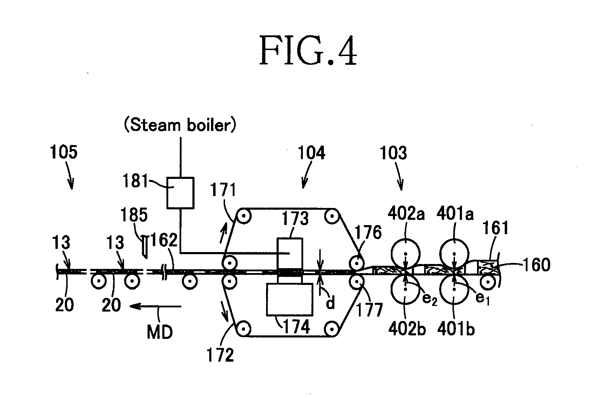

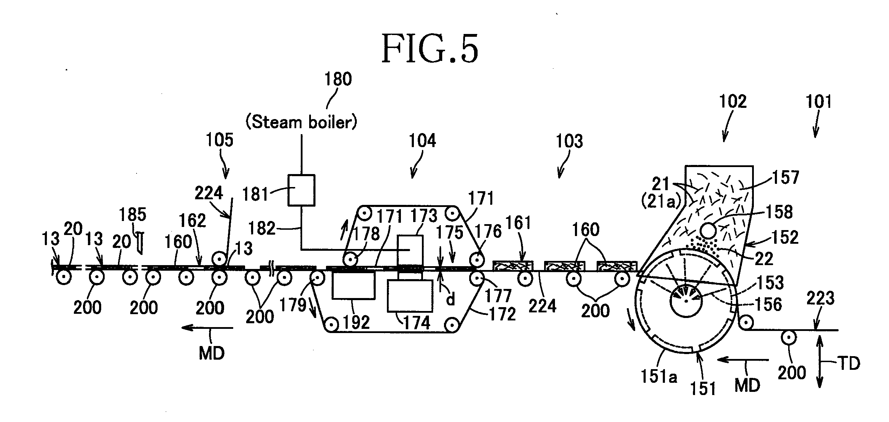

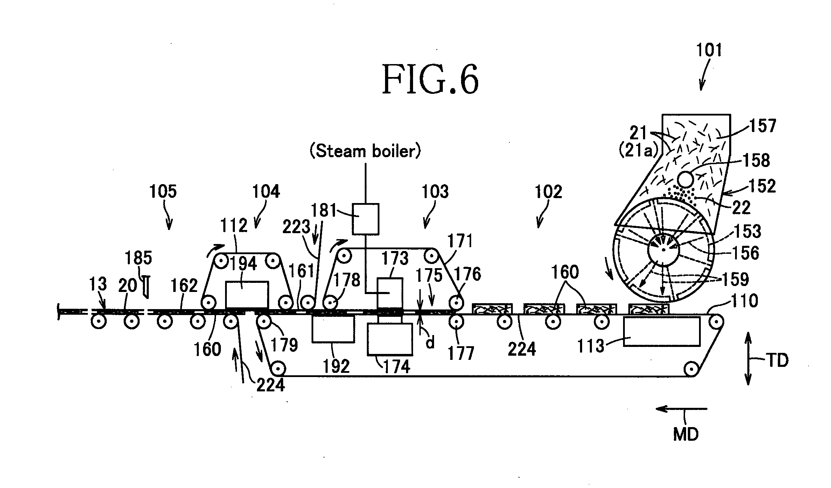

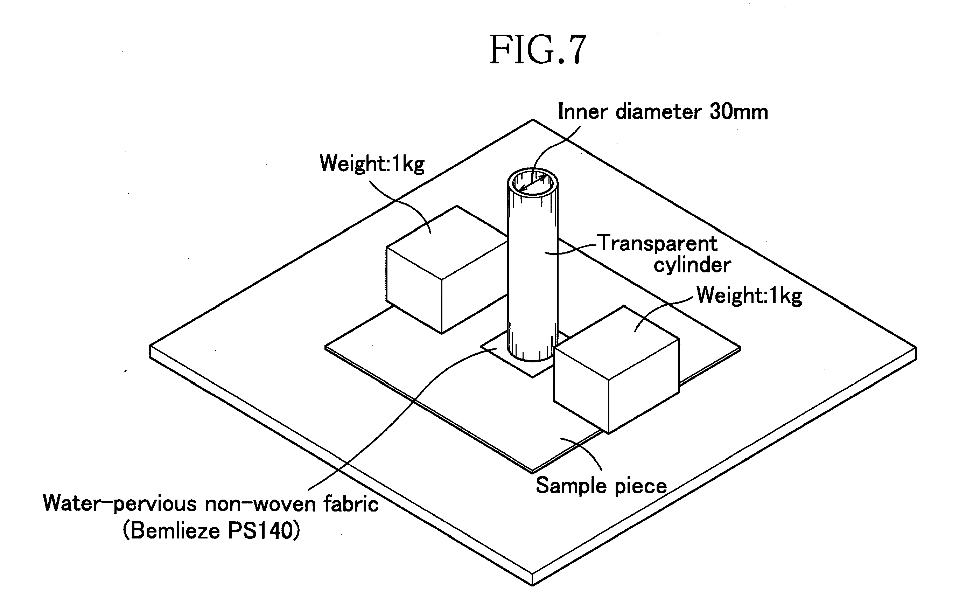

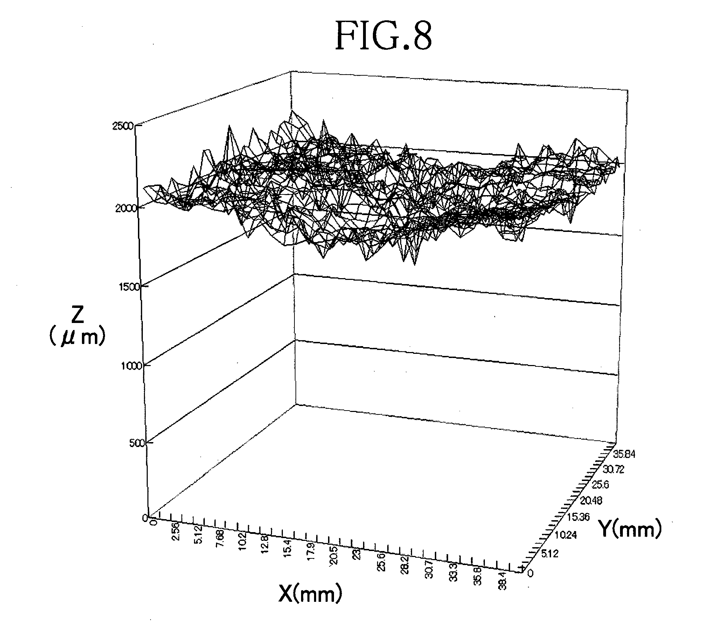

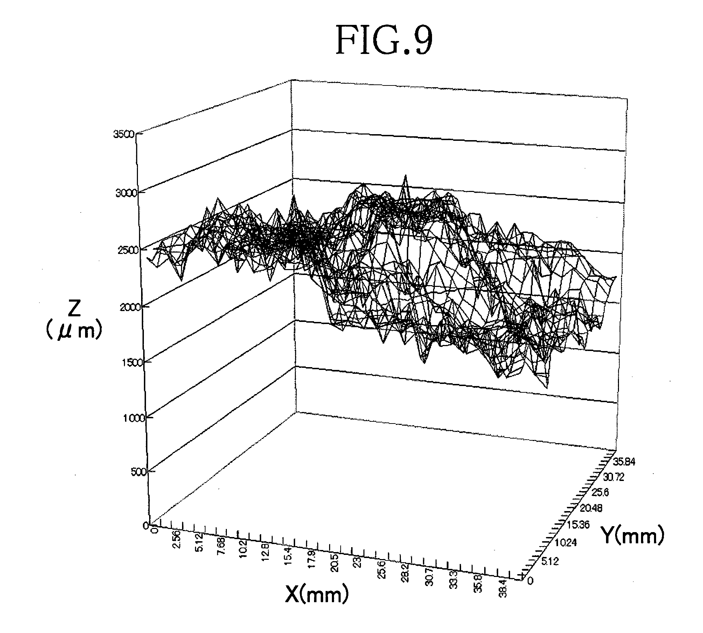

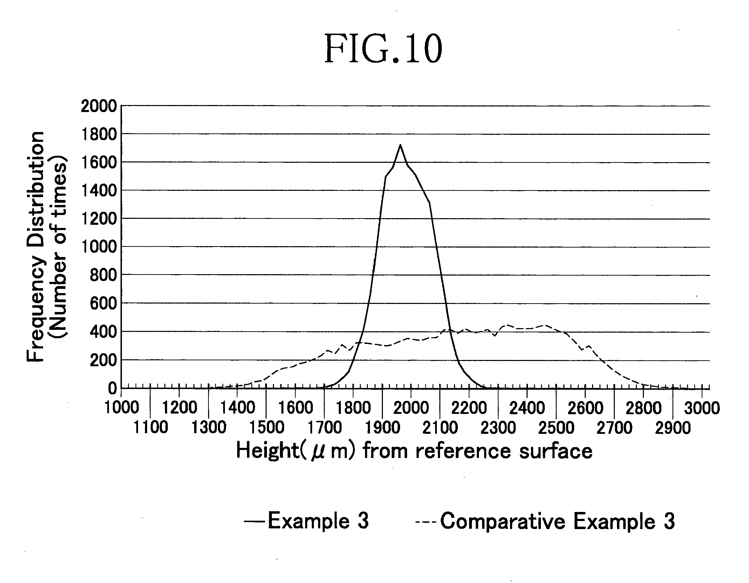

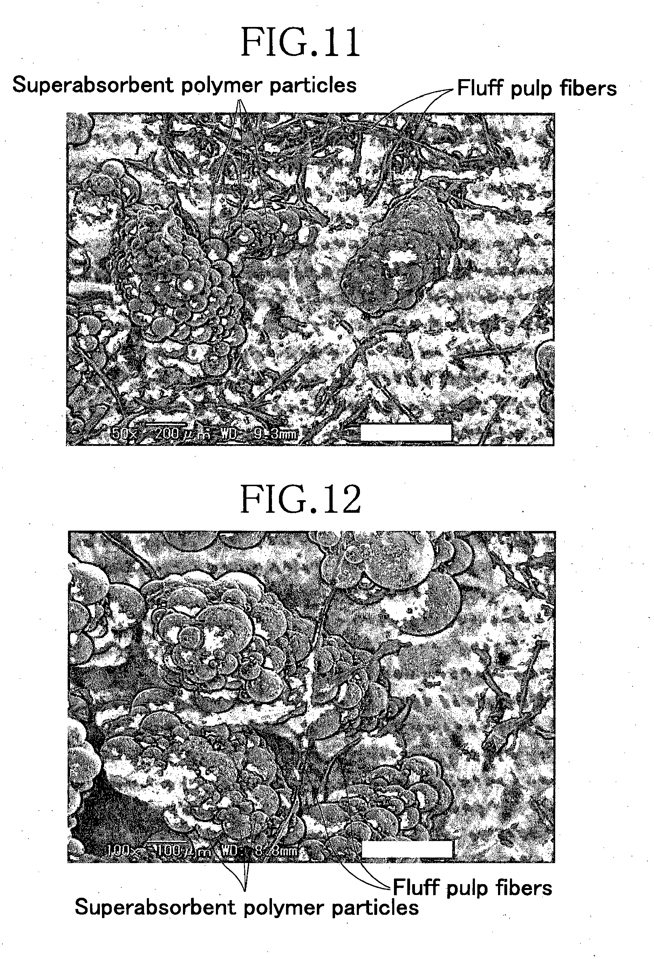

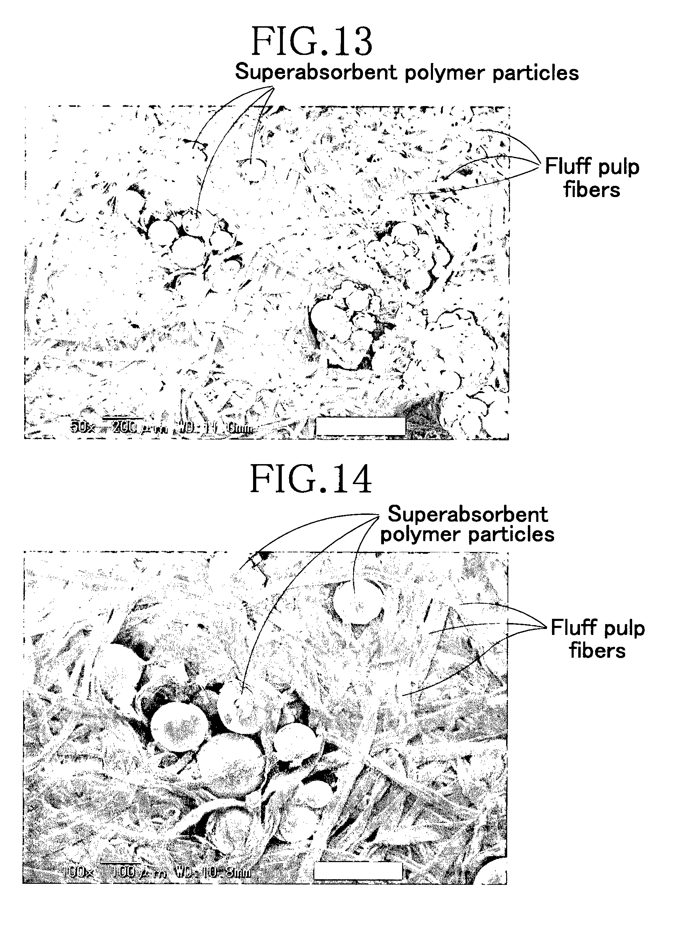

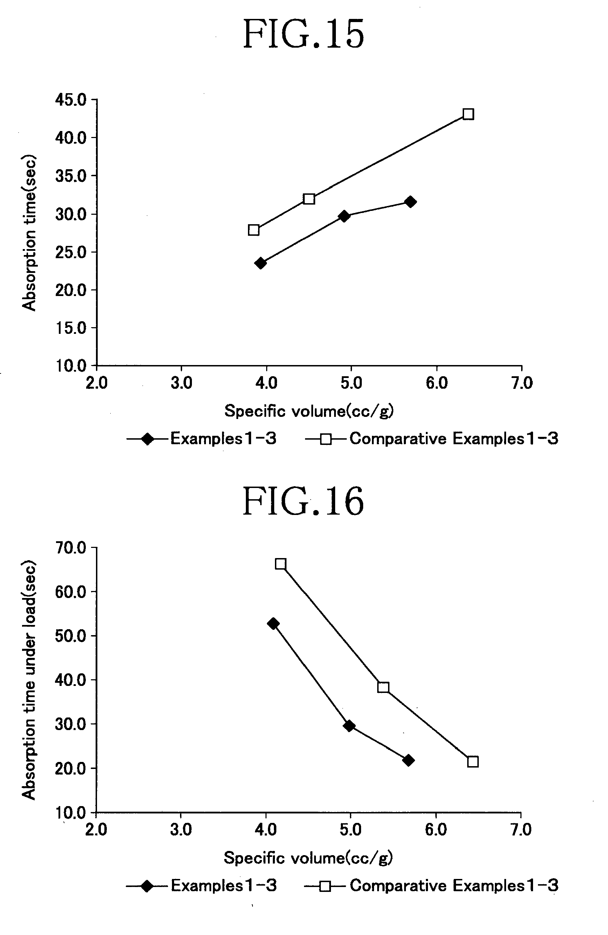

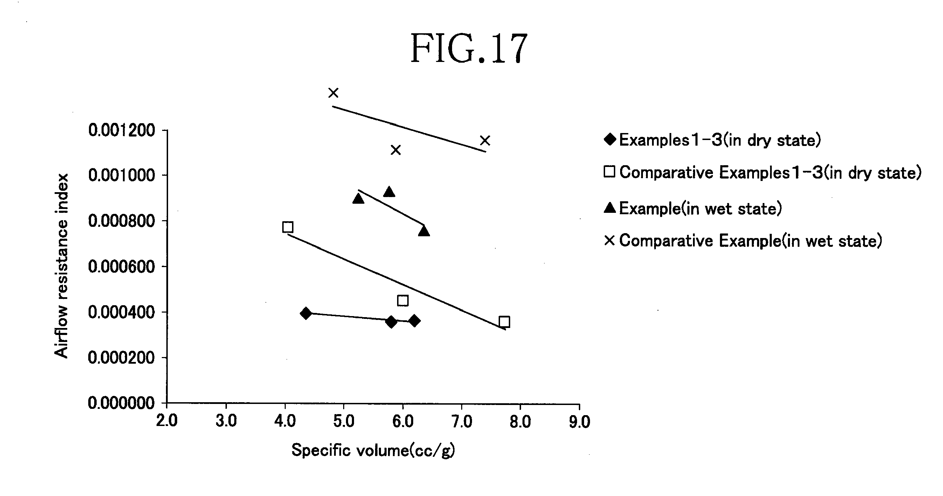

{Fig. 1} Fig. 1 is a partially cutaway plan view of a disposable diaper.
{Fig. 2} Fig. 2 is a sectional view taken along the line II-II in Fig. 1.
{Fig. 3} Fig. 3 is a diagram illustrating one embodiment of a process for making a core including an aggregate of water-absorbent materials.
{Fig. 4} Fig. 4 is a diagram illustrating another embodiment of the process for making the core.
{Fig. 5} Fig. 5 is a diagram illustrating still another embodiment of the process for making the core.
{Fig. 6} Fig. 6 is a diagram illustrating yet another embodiment of the process for making the core.
{Fig. 7} Fig. 7 is a diagram illustrating a measuring method for absorption rate under a load.
{Fig. 8} Fig. 8 is a 3D graphic diagram illustrating a surface undulation in the aggregate of water-absorbent materials according to an Example of the present invention.
{Fig. 9} Fig. 9 is a 3D graphic diagram illustrating a surface undulation in the aggregate of water-absorbent materials according to a Comparative Example.
{Fig. 10} Fig. 10 is a graphic diagram comparatively illustrating a height distribution from a base level to a top surface of the aggregate of water-absorbent materials in the Example and Comparative Example.
{Fig. 11} Fig. 11 is a photo of the water-absorbent material aggregate's surface in the Example taken at 50-fold magnification;
{Fig. 12} Fig. 12 is a photo of the water-absorbent material aggregate's surface in the Example taken at 100-fold magnification.
{Fig. 13} Fig. 13 is a photo of the water-absorbent material aggregate's surface in the Comparative Example taken at 50-fold magnification.
{Fig. 14} Fig. 14 is a photo of the water-absorbent material aggregate's surface in the Comparative Example taken at 100-fold magnification.
{Fig. 15} Fig. 15 is a graphic diagram plotting relationships between absorption rates and specific volumes.
{Fig. 16} Fig. 16 is a graphic diagram plotting relationships between absorption rates under a load and specific volumes.
{Fig. 17} Fig. 17 is a graphic diagram plotting relationships between airflow resistive indices and specific volumes.
{Fig. 18} Fig. 18 is a diagram exemplarily illustrating a process of the prior art for making a core.

### {Description of Embodiments}

Details of the method for reducing a thickness of an aggregate of water-absorbent materials according to the present invention and the aggregate of water-absorbent materials sufficiently thinned using this method will be described with reference to the accompanying drawings.

Fig. 1 is a partially cutaway plan view of an open-type disposable diaper 1 using a thin water-absorbent material aggregate according to one embodiment of the present invention as a bodily fluid absorbent core 13. The diaper 1 has a front-back direction A and transverse direction B extending orthogonally to each other and includes a rectangular chassis 2 which is relatively long in the front-back direction A, a pair of front wings 3 attached to a front portion of the chassis 2 to extend in the transverse direction B and a pair of rear wings 4 attached to a rear portion of the chassis 2 to extend in the transverse direction B. In the front-back direction A of the chassis 2, a crotch region 6 is defined between the front wings 3 and the rear wings 4, a front waist region 7 is defined in front of the crotch region 6 and a rear waist region 8 is defined behind the crotch region 6.

The chassis 2 includes a liquid-pervious topsheet 11, a liquid-impervious backsheet 12 and a bodily fluid absorbent core 13 sandwiched between these top- and backsheets 11, 12 wherein the backsheet 12 is covered with an outer sheet 14 made of nonwoven fabric providing comfortable texture. The topsheet 11 and the backsheet 12 extend outward beyond a peripheral edge 51 of the core 13 and are put flat and bonded together with hot melt adhesive 41a outside the peripheral edge 51. Portions of these topsheet 11, backsheet 12 and outer sheet 14 extending outward beyond the peripheral edge 51 of the core 13 respectively define opposite side edges 18 and front and rear ends 61, 62. The side edges 18 are respectively provided with leak barriers 31 formed of pieces of sheet which are relatively long in the front-back direction A. Each of the leak barriers 31 has a basal edge 33 bonded to the associated side edge 18 with hot melt adhesive 32a, a front end 34 bonded to the front end 61 with hot melt adhesive 32b, a rear end 36 bonded to the rear end 62 with hot melt adhesive 32c and a free edge 37 opposed to the associated basal edge 33 and disposed inwardly as viewed in the transverse direction of the chassis 2 to overlap the topsheet 11 and adapted to be spaced upward from the topsheet 11. The free edge 37 is formed with a sleeve 38 containing an elastic member 39 bonded under tension to an inner surface thereof with hot melt adhesive (not shown).

The side edges 18 of the chassis 2 are respectively further provided with leg elastic members 41 sandwiched between the outer sheet 14 and the respective basal edges 33 of the leak barriers 31 to extend in the front-back direction A and attached under tension to the outer sheet 14 with hot melt adhesive 41a. The front end 61 of the chassis 2 is provided with a front waist region elastic member 42 sandwiched between the topsheet 11 and the backsheet 12 to extend in the transverse direction B and attached under tension to at least one of these top- and backsheets 11, 12 with hot melt adhesive (not shown). The rear end 62 of the chassis 2 is provided with a rear waist region elastic member 43 sandwiched between the topsheet 11 and the backsheet 12 to extend in the transverse direction B and attached under tension to at least one of these top- and backsheets 11, 12 with hot melt adhesive (not shown).

The chassis 2 constructed in this manner is provided along the side edges 18 in the front waist region 7 with the front wings 3 respectively extending outward in the transverse direction B and along the side edges 18 in the rear waist region 8 with the rear wings 4 respectively extending outward in the transverse direction B. The rear wings 4 are respectively provided with tape fasteners 46. These tape fasteners 46 are adapted to be unfolded in the transverse direction B as indicated by imaginary lines and to be temporarily fixed to the outer surface of the chassis 2 or the outer surfaces of the respective front wings 3 with pressure-sensitive adhesive 47 applied to inner surfaces of the respective tape fasteners 46.

Fig. 2 is a sectional view taken along the line II-II in Fig. 1 wherein a thickness direction of the core 13 is indicated by the double-headed arrow C. The line II-II corresponds to the center line M-M bisecting a dimension of the chassis 2 in the front-back direction A. The core 13 includes a compressed aggregate 20 which has been formed by compressing water-absorbent materials including hydrophilic fibers 21 and superabsorbent discrete polymer particles 22 until the aggregate 20 becomes sufficiently thin, air-permeable and liquid-pervious upper sheet 23 and air-permeable lower sheet 24 covering upper and lower surfaces of the compressed aggregate 20, respectively.

Structural proportion of the hydrophilic fibers 21 in the compressed aggregate 20 is in a range of 98 to 10% by mass and, as the hydrophilic fiber 21, for example, natural fiber such as fluff pulp fibers or cotton fibers, semisynthetic fibers such as rayon fibers, or thermoplastic synthetic fibers modified to become hydrophilic each having a fiber length in a range of 2 to 80mm may be used. It should be understood here that 15% or less by mass of the compressed aggregate 20 may be replaced by hydrophobic thermoplastic synthetic fibers not modified to become hydrophilic and having a fiber length in a range of 20 to 80mm. Such thermoplastic synthetic fibers may sometimes promote bodily fluids to spread in the compressed aggregate 20.

The proportion of the superabsorbent polymer particles 22 in the structure of the compressed aggregate 20 is in a range of 2 to 90% by mass and, as the superabsorbent polymer particles 22, polymer particles commonly used in the related technical field such as particles of polyacrylic acid, polyacrylate, starch-acrylonitrile graft copolymer, polyvinyl alcohol, polyvinyl ether, polyacrylamide, carboxymethyl cellulose or natural polysaccharide can be used. While particles 22 of these polymers may have spherical or fibrous shape and may sometimes be amorphous, the present invention is not limited to any particular shape of the polymer particles 22 and the polymer particles 22 of any shape can be used as the superabsorbent polymer particles 22 so far as the polymer particles 22 can be mixed with the hydrophilic fibers 21. In other words, the superabsorbent polymer particles 22 according to the present invention may be defined as "superabsorbent polymer particles having the shape adapted to be mixed with the hydrophilic fibers 21". It should be appreciated here that the expression "the superabsorbent polymer particles 22 can be mixed with the hydrophilic fibers 21" used herein includes, in addition to the above-mentioned case in which the superabsorbent polymer particles 22 can be uniformly mixed with the hydrophilic fibers 21, the case in which the superabsorbent polymer particles 22 are eccentrically-located in the compressed aggregate 20 of the water-absorbent materials, in other words, in any region of the core 13 of Fig. 2, in the front-back direction A, the transverse direction B or the thickness direction C.

The upper sheet 23 is used to face the lower surface of the topsheet 11 and the lower sheet 24 is used to face the upper surface of the backsheet 12. As the upper sheet 23 and the lower sheet 24, a tissue paper having a basis mass in a range of 10 to 30g/m² or a nonwoven fabric having a basis mass in a range of 5 to 40g/m² may be used. More specifically, air-permeable sheet materials or, in addition, liquid-pervious sheet materials both adapted to facilitate permeation of water vapor as will be described later may be preferably used. For example, it is required for the upper sheet 23 to be air-permeable and liquid-pervious so that bodily fluids may smoothly move from the topsheet 11 to the compressed aggregate 20. It is required for the lower sheet 24 also to be air-permeable and liquid-pervious and sometimes required to be less liquid-pervious than the upper sheet or liquid-impervious so that movement of bodily fluids from the compressed aggregate 20 to the backsheet 12 may be restricted. The upper sheet 23 and the lower sheet 24 extend outward beyond the peripheral edge of the compressed aggregate 20 and put flat and bonded together outside the peripheral edge and thereby serve to maintain the initial shape of the compressed aggregate 20.

Fig. 3 is a diagram exemplarily illustrating a process according to the present invention for making the compressed aggregate 20 and the core 13 including this aggregate 20. In Fig. 3, the machine direction MD in which various kinds of material are transported and a thickness direction TD corresponding to the thickness direction C of the core 13 and extending orthogonally to the machine direction MD are indicated by arrows. A direction orthogonal to both the machine direction MD and the thickness direction TD is a cross direction CD (not shown). The process illustrated in Fig. 3 includes first through fifth steps 101 to 105. In the first step 101 located upstream in the machine direction MD, an air-permeable or an air-permeable and liquid-pervious second sheet web 224 corresponding to a continuous lower sheet 24 is continuously fed in the machine direction MD by delivery rolls 200 rotating in the machine direction MD.

The second step 102 includes a suction drum 151 rotating in the machine direction MD and a hooded water-absorbent material feeding region 152 to cover the suction drum 151. On a peripheral surface 151a of the suction drum 151 is formed a plurality of depressions 153 each having a shape substantially corresponding to a planar shape of the core 13 and arranged at a predetermined pitch in a peripheral direction. During rotation of the suction drum 151, the depression 153 is subjected to vacuum suction effect 156 as this depression 153 reaches the feeding region 152. The feeding region 152 is the region in which the water-absorbent materials according to the present invention is delivered to the suction drum 151 and includes a fluff pulp fiber feeding region 157 adapted to feed the fluff pulp fibers 21a as the hydrophilic fibers 21 constituting the water-absorbent materials and a superabsorbent polymer particle feeding region 158 adapted to feed the superabsorbent polymer particles 22 constituting the water-absorbent materials. With such combination, the feeding region 152 is adapted to feed the fluff pulp fibers 21a and the superabsorbent polymer particles 22 into the depression 153 reaching the feeding region 152 so that the fluff pulp fibers 21a and the superabsorbent polymer particles 22 may be mixed or laminated with each other. The second sheet web 224 coming from the first step 101 is received by the peripheral surface 151a of the suction drum 151, then reaches the feeding region 152 and is successively deformed in accordance with the shapes of the respective depressions 153 under the vacuum suction effect 156. In this way, the second sheet web 224 covers the surfaces of the respective depressions 153. The fluff pulp fibers 21a and the superabsorbent polymer particles 22 are delivered into the depressions 153 after these depressions 153 have been covered with the second sheet web 224 in this manner. While the hooded feeding region 152 is substantially implemented in the form of a closed structure, a clearance is left between the feeding region 152 and the peripheral surface 151a of the suction drum 151 so that the second sheet web 224 and a water-absorbent material aggregate 160 as will be described later may smoothly run forward.

In the third step 103, the second sheet web 224 leaving the peripheral surface 151a of the suction drum 151 is transported forward by delivery rolls 200 in the machine direction MD. On the second sheet web 224, a plurality of the aggregates 160 of water-absorbent material each deformed in accordance with the planar shape of the depression 153 and in a still-not-compressed state are arranged intermittently in the machine direction MD. Each of the aggregates 160 includes the fluff pulp fibers 21a and the superabsorbent polymer particles 22 delivered into the depression 153 and accumulated therein in the second step 102. In the third step 103, an air-permeable or, in addition, liquid-pervious first sheet web 223 comprising continuously arranged upper sheets 23 is continuously fed from above as viewed in the diagram and cooperates with the second sheet web 224 to sandwich the aggregates 130 therebetween. In this way, these first and second sheet webs 223, 224 cooperate with the aggregates 160 arranged intermittently in the machine direction MD to form a first composite web 161.

The fourth step 104 in Fig. 3 includes air-permeable first and second mesh conveyor belts 171, 172 vertically paired as viewed in Fig 3, a steam ejection unit 173 and a steam suction unit 174. The vertically paired first and second mesh conveyor belts 171, 172 are air-permeable support means for the aggregates 160 and for the first composite web 161 serving to transport the first composite web 161 including the aggregates 160 in the machine direction MD under compressive effect in the thickness direction of the aggregates 160, i.e., in the thickness direction TD in Fig. 3. Along parallel running segments 175 adapted to support the aggregates, the first and second mesh conveyor belts 171, 172 run in parallel to each other, for example, at a velocity in a range of 5 to 500m/min in the machine direction MD. A nip between an upper roll 176 and a lower roll 177 on the upstream side, both rotating in the machine direction MD, as well as a nip between an upper roll 178 and a lower roll 179 on the downstream side, both rotating in the machine direction MD, may be adjusted to adjust a clearance d between the parallel running segments 175 in the thickness direction TD. In the fourth step 104, the aggregates 160 and the first composite web 161 can be compressed to a desired thickness by these first and second mesh conveyor belts 171, 172 of which the parallel running segments 175 are adjustably spaced from each other in the thickness direction TD. The parallel running segments 175 cooperate with the steam ejection unit 173 and the steam suction unit 174 opposed to each other about the parallel running segments 175 of the first and second mesh conveyor belts 171, 172. The steam ejection unit 173 includes a plurality of nozzles (not shown) each having a diameter, for example, in a range of 0.1 to 2mm arranged in the cross direction CD (not shown) extending orthogonally to the machine direction MD and to the thickness direction TD at a pitch preferably in a range of 0.5 to 10mm, more preferably in a range of 0.5 to 5mm and most preferably in a range of 0.5 to 3mm to extend across the first composite web 161. The respective nozzles are supplied via a piping 182 with steam at a temperature corresponding to water's boiling point or higher generated within a steam boiler 180 and regulated by a pressure-regulating valve to a steam pressure, for example, in a range of 0.1 to 2.0MPa. Such high pressure steam streams(not shown) are ejected from the respective nozzles against the first composite web 161 compressed by the first and second mesh conveyor belts 171, 172 through the first mesh conveyor belt 171. Ejection quantity of steam streams against the aggregates 160 contained in the first composite web 161 is regulated depending on the running velocity of the first and second mesh conveyor belts 171, 172. Assuming that the first and second mesh conveyor belts 171, 172 run at a velocity in a range of 5 to 500m/min, steam streams in a range of 1.23kg/m² to 0.03kg/m² per unit area of the aggregates facing the first mesh conveyor belt 171 are preferably ejected against the aggregates 160 through the first sheet web 223. Steam streams pass through the first mesh conveyor belt 171, the first composite web 161 and the second mesh conveyor belt 172 in this order in the thickness direction C of the aggregate 160 and is recovered under the vacuum suction effect of a steam suction unit 174. The first composite web 161 against which steam has been ejected leaves the first and second mesh conveyor belts 171, 172 in the machine direction MD and runs forward to the fifth step 105 in the form of second composite web 162.

In this fourth step 104, at least one of the first and second mesh conveyor belts 171, 172 may be made of material having a sufficient flexibility to be easily deformed in the thickness direction TD to prevent the first composite web 161 from being locally compressed by these first and second mesh conveyor belts 171, 172. Specifically, as the first and second mesh conveyor belts 171, 172, metallic wire mesh belts formed of, for example, stainless alloy or bronze or plastic mesh conveyor belts formed, for example, of polyester fiber or aramid fiber may be used. It is also possible to use a metallic belt formed of a perforate metal plate. For the application in which it is essential to prevent metal powder from getting mixed into the aggregate and the other components, the plastic mesh conveyor belts may preferably be used. For the application in which the plastic mesh conveyor belts are preferably used and high heat resistance is required for the plastic mesh conveyor belts, the mesh belts made of polyphenylene sulfide resin may be preferably used. Plain woven mesh belts of 10 to 75 meshes are flexible and is one example of a particularly preferable mesh belt which may be used for the first mesh conveyor belt 171 and for the second mesh conveyor belt 172. The steam ejection unit 173 and the piping 182 may be preferably provided with appropriate heat-retention means and draining mechanism. Such countermeasures may prevent apprehension that drainage generated within the steam ejection unit 173 or the other units might be ejected from the nozzles and make the first composite web 16 have an excessively wetted state and/or damage the first sheet web 223 when the latter is formed of a tissue paper. The steam streams may be ejected against the first composite web 161 in the form of dry steam containing no moisture, saturated steam or wet steam containing moisture. Wet steam or saturated steam can easily make the hydrophilic fibers 21 into a wet state and thereby easily deform the hydrophilic fibers 21. Dry steam can gasify the moisture contained in the fluff pulp fibers 21a, if the hydrophilic fibers 21 are fluff pulp fibers 21a and the moisture gasified in this manner facilitates the hydrophilic fibers 21 to be deformed. In the hydrophilic fibers 21 comprising thermoplastic synthetic fibers, heat of the dry steam facilitates the thermoplastic synthetic fibers to be deformed. In the steam ejection unit 173 provided with the heating mechanism, the steam streams can be ejected in the form of overheated steam. The steam suction unit 174 is preferably provided with the piping by which the sucked high pressure steam may be guided to an exhaust blower (not shown) after the high pressure steam has passed through a steam-water separator. It should be appreciated here that the present invention may be implemented in a manner that the steam ejection unit 173 and the steam suction unit 174 in the fourth step 104 may be positionally interchanged, i.e., the steam ejection unit 173 may lie on the downside of the steam suction unit 174. If it is unnecessary, in the fourth step 104, to collect the high pressure steam having passed through the first composite web 161, the present invention may be implemented without setting up the steam suction unit 174.

In the fifth step 105, the second composite web 162 having left behind the first and second mesh conveyor belts 171, 172 is received by the deliver rolls 200 to run in the machine direction MD and, in the course of running, cut along a middle line between each pair of the adjacent aggregates 160 into the individual core 13. The aggregate 160 obtained in this manner is an aggregate 20 (See Fig. 2) compressed by the first and second mesh conveyor belts 171, 172 and the steam wherein the first and second sheet webs 223, 224 define the upper and lower sheets 23, 24 of the core 13. Though not shown, in the fifth step 105, it is possible to roll up the second composite web 162 in the form of a roll comprising a continuum of the cores 13 and to provide this continuum to a production line of disposable diapers. It is possible to include a dryer device for the second composite web 162.

In the compressed aggregate 20 in the core 13 shown in Fig. 2 obtained by the process of Fig. 3, the aggregate 160 of water-absorbent material including a mixture of the hydrophilic fibers 21 and the superabsorbent polymer particles 22 has been compressed between the first mesh conveyor belt 171 and the second mesh conveyor belt 172 under ejection of higher pressure steam streams. Consequently, the hydrophilic fibers 21, for example, the fluff pulp fibers 21a are quickly deformed in a heated and humidified state or a simply heated state and lose any repulsive power against the compressive force of the first and second mesh conveyor belts 171, 172. As a result, the fluff pulp fibers 21a may maintain a shape which is substantially the same as the shape immediately after compression. Moisture contained in the steam facilitates each pair of the adjacent hydrophilic fibers 21 to get closer to each other and an interaction between each pair of closely adjacent hydrophilic fibers facilitates the respective hydrophilic fibers to maintain their respective shapes immediately after having been compressed. In addition, compared to the prior art according to which the first composite web 161 is sprayed with water and then the first composite web 161 is compressed by the compression rolls or the like, the high pressure steam streams used by the present invention passes through the first composite web 161 more smoothly than sprayed water and therefore the aggregate 160 can be quickly brought to a heated and a humidified state fully in the thickness direction of the aggregate 160. In the process of Fig. 3, the clearance d between the first and second mesh conveyor belts 171, 172 may be set to a value required for the second composite web 162 to obtain the second composite web 162 having a desired thickness without setting the clearance d to an excessively small value.

A ratio of the second composite web 162 versus the clearance d is a value indicating a recovery rate r of the first composite web 161 after compression and this recovery rate r represents a degree of the effect of the high pressure steam used in the fourth step 104 for compression of the first composite web 16. The recovery rate r which is approximate to 1 means that the thickness of the second composite web 162 is substantially equal to the clearance d. According to the present invention using the fourth step 104, the recovery rate r is often approximately 1. When the first composite web 161 is compressed merely by the compression roll pair or the first composite web 161 is thinned by the compression roll pair after water has been sprayed onto the first composite web as has been the case in the prior art, the recovery rate r often has a value largely surpassing 1. In the prior art, for example, intending to thin the first composite web 161 to the desired thickness merely by using the compression roll pair, it is required to set the clearance d to a value much smaller than the desired thickness. In view of such significant difference between the present invention and the prior art, it is possible, according to the present invention, to prevent the problem that the superabsorbent polymer particles 22 might partially or wholly collapse due to excessive compression of the aggregate 160 of water-absorbent material and the first composite web 161.

According to the present invention, by employing the process illustrated in Fig. 3, in which the high pressure steam and the flexible first and second mesh conveyor belts 171, 172 are used to compress the first composite web 161, both the compressive effect of the steam and the compressive effect of the first and second mesh conveyor belts 171, 172 would not be locally concentrated even if the aggregate 160 of water-absorbent materials has regions locally thicker than the remaining regions. For example, assuming that the aggregate 160 has a region in which the superabsorbent polymer particles 22 are eccentrically located and the thickness of the aggregate 160 is relatively thick due to the presence of the eccentrically located superabsorbent polymer particles 22, if the first composite web 161 including such aggregate 160 of water-absorbent materials is compressed by the compression roll pair as has been the case in the prior art, the compressive force of the compression roll pair might be concentrated on such thicker region due the presence of the super absorbent polymer particles 22, causing the superabsorbent polymer particles 22 in such region to partially or wholly collapse and/or putting the superabsorbent polymer particles 22 and the hydrophilic fiber 21 in undesirably close contact to one another and/or putting the hydrophilic fibers 21 themselves in excessively close contact with one another. In contrast with this, when the first composite web 161 is moderately compressed by the first and second mesh conveyor belts 171, 172 under the effect of steam streams ejections as in the present invention, it is possible to restrict a compressive force from being concentrated on the relatively thick region of the first composite web 161. Consequently, it is possible to prevent partial or whole collapse of the superabsorbent polymer particles 22 from occurring and thereby it is possible to obtain the flexible cores 13 which are sufficiently thin but exhibit a high absorption rate.

Assuming that the superabsorbent polymer particles 22 have spherical shapes, collapse thereof may lead to exposure of polymer components within the particles which have low crosslink density and thereby the superabsorbent polymer particles having absorbed water may readily form a gel block. As a consequence, there is substantially no chance that the superabsorbent polymer particles 22 which are included within the gel block come into contact with bodily fluids and cannot function as the water-absorbent materials. In addition, formation of the gel block transforms the superabsorbent polymer particles 22 from those of small diameter to those of larger diameter. As a result, formation of the gel block deteriorates the primary water absorbing ability and/or the desired flexibility of the aggregate 160. On account of this, the collapse of the superabsorbent polymer particles 22 in the bodily fluid absorbent core 13 should be preferably restricted. In addition, a phenomenon that the superabsorbent polymer particles 22 come in close contact with the hydrophilic fibers 21, and surfaces of the respective particles 22 are covered with the fibers 21 also should be preferably restricted because the fibers 21 covering the surfaces of the particles 22 in this manner prevent the particles 22 from coming in contact with bodily fluids and make it difficult for the particles 22 to absorb water quickly and eventually the super absorbent polymer particles 22 in the core material 13 may not function quickly. Further, close contact between each pair of the adjacent hydrophilic fibers 21 also should be preferably restricted because such close contact delays permeation of bodily fluids through interstices of the fibers and deteriorates the water absorption rate of the aggregate 160.

The process of Fig. 3 according to the present invention may be partially modified. For example, it is possible to place the first composite web 161 not on the delivery rolls 200 but on a conveyor belt to deliver it to the fourth step 104. It is also possible to place the second composite web 162 not on the delivery rolls 200 but on a conveyor belt to feed it in the machine direction MD. In the first step 101 of the process illustrated in Fig. 3, it is possible to feed not the second sheet web 224 but the first sheet web 223 in the machine direction MD and, in the third step 103, it is possible to feed not the first sheet web 223 but the second sheet web 224 in the machine direction MD. While the second sheet web 224 and the first and second composite webs 161, 162 run horizontally in the machine direction MD in the first step 101 and the third, fourth and fifth steps 103, 104, 105 in the illustrated embodiment, it is possible to arrange it so that the paths for the second sheet web 224 and the first and second composite webs 161, 162 may be modified to extend in vertical or tilted directions. Such modifications may be appropriately selected depending on various factors such as a plant space available for the process of Fig. 3.

Fig. 4 is a diagram similar to Fig. 3 except for the third step 103, illustrating another embodiment of the present invention. The third step 103 in Fig. 4 includes a first compression roll pair 401a, 401b and a second compression roll pair 402a, 402b for the purpose of moderately compressing the first composite web 161 by mechanical means. A roll nip e₁ of the first compression roll pair 401a, 401b and a roll nip e₂ of the second compression roll pair 402a, 402b are set to compress the first composite web 161 to a degree of thickness with which the first composite web 161 can smoothly make its way into the respective nips of the first and second mesh conveyor belts 171, 172. The lower limits of the nips e₁, e₂ are larger than a nip between the upper roll 176 and the lower roll 177 and thereby function to prevent the first composite web 161 from being excessively compressed by the first compression roll pair 401a, 401b and the second compression roll pair 402a, 402b and thereby function to prevent the superabsorbent polymer particles 22 contained in the aggregate 160 from partially or wholly collapsing. Usually, the first composite web 161 having left behind the second step 102 of the process of Fig. 3 is apt to become bulky in proportion to a content rate of the hydrophilic fibers 21 and, when the bulky first composite web 161 makes its way into the nips of the first and second mesh conveyor belts 171, 172 both having relatively small clearances d, the aggregate 160 of water-absorbent materials may be distorted under a force functioning to thrust the aggregate 160 back upstream in the machine direction MD. However, in the third step 103 in the process of Fig. 4, the shape distortion or other abnormal situation which would otherwise occur in the aggregate 160 when the first composite web 161 makes its way into the nips of the first and second mesh conveyor belts 171, 172 after having been compressed by the first compression roll pairs 401a, 401b and the second compression roll pairs 402a, 402b is effectively prevented and the sufficiently thin core 13 can be produced.

The third step 103 of Fig. 4 may be variously modified. For example, any one of the first compression roll pair 401a, 401b and the second compression roll pair 402a, 402b may be eliminated. Furthermore, the third step 103 may include one or more additional compression roll pair(s).

Fig. 5 is a diagram illustrating still another embodiment of the present invention. In the first step 101 in the process of Fig. 5, the air-permeable first sheet web 223 is continuously fed in the machine direction MD. In the third step 103 in the process of Fig. 5, the first composite web 161 includes the first sheet web 223 and the aggregates 160 of water-absorbent material intermittently placed on the first sheet web 223 and does not include the second sheet web 224 in Fig. 3. In the fourth step 104, such first composite web 161 is introduced between the first and second mesh conveyor belts 171, 172 opposed to each other with the clearance d and simultaneously steam streams are ejected from the steam ejection unit 173 to the aggregates 160. Conditions of such steam ejections and the suction thereof are the same as the conditions employed in the process of Fig. 3. It should be appreciated here that the length of the first mesh conveyor belt 171 along its parallel running segment is shorter than the length of the second mesh conveyor belt 172. The fourth step 104 includes a second suction box 192 provided below the first composite web 161 and the second mesh conveyor belt 172 in the vicinity of the upper roll 178 on a downstream side at which the first mesh conveyor belt 171 running in the machine direction MD is separated from the first composite web 161. Vacuum suction force exerted from the second suction box 192 upon the aggregates 160 through the second mesh conveyor belt 172 functions to prevent fibers of the aggregates 160 which have been covered with the first mesh conveyor belt 171 from fluffing and/or flying apart. After the first composite web 161 has left behind the fourth step 104, the surfaces of the respective aggregates 161 having faced the first mesh conveyor belt 171 are now covered with the second sheet web 224 in the fifth step 105 to form the second composite web 162. The second composite web 162 is intermittently cut by the cutter 185 into the individual cores 13 and the aggregates 160 are divided into the individual compressed aggregates 20.

In this manner, the process of Fig. 5 may use the same hydrophilic fibers 21, the superabsorbent polymer particles 22 and the first sheet web 223 as used in the process of Fig. 3. While it is also possible to use the same second sheet web 224 as that used in the process of Fig. 3, not only the air-permeable sheet web but also an air-nonpermeable sheet web or an air-and-liquid-nonpermeable sheet may be used because no steam ejections through the second sheet web 224 occurs in the process of Fig. 5. The core 13 obtained using the liquid-impervious second sheet web 224 may be used in a manner that the second sheet web 224 defines the leak-barrier backsheet in the bodily fluid absorbent disposable article such as a disposable diaper or a menstruation napkin. In the process of Fig. 5, when an air-permeable sheet web is used as the first sheet web 223 and the sheet web 224, the process of Fig. 5 may be modified so that the second sheet web 224 is fed in the first step 101 and the first sheet web 223 is fed in the fifth step 105.

Fig. 6 is a diagram similar to Fig. 5, illustrating yet another embodiment of the present invention. The process of Fig. 6 also includes first through fifth steps 101 through 105. The first step 101 includes an air-permeable endless belt 110 adapted to run in the machine direction MD, the suction drum 151 and the hooded water-absorbent material feeding region 152 provided above the air-permeable endless belt 110. While both the drum 151 and the feeding region 152 are the same as those exemplarily illustrated in Fig. 5, the second sheet web 224 is not delivered to the drum 151. The drum 151 is formed on its peripheral surface with the depressions 153 at a predetermined pitch in the circumferential direction. Each of the depressions 153 is subjected to the suction effect as the depression 153 makes its way into the feeding region 152. In the feeding region 152, the hydrophilic fibers 21 such as fluff pulp fibers and the superabsorbent polymer particles 22 move towards the depression 153 and, in consequence, the aggregate 160 of water-absorbent material is formed in accordance with the shapes of the depressions 153. When the drum 151 rotates counterclockwise and the aggregate 160 moves to a position immediately above the belt 110, the depression 153 is subjected not to the suction effect but to the blower and the aggregates 160 are discharged onto the belt 110. Below the drum 151, the third suction box 113 is located via the belt 110 and the aggregates 160 having been discharged are received by the belt 110 under the suction effect of the third suction box 113. On the drum, in addition to or instead of using the blower 159, an appropriate mechanical effect may be used to retract the bottom of the depression 153 in the radial direction and to discharge the aggregates 160.

In the second step 102 of Fig. 6, the aggregates 160 are placed on the belt 110 and run in the machine direction MD. The belt 110 runs, for example, at a rate of 5 to 500m/min toward the third step 103.

The third step 103 in the process of Fig. 6 includes, in addition to the belt 110, the first mesh conveyor belt 171, the steam ejection unit 173, the steam suction unit 174 and the second suction box 192, the same as those used in Fig. 3 or Fig. 5. To set the clearance d between the belt 110 and the first mesh conveyor belt 171 to the desired value, a clearance in the vertical direction between the upper roll of upstream 176 and the lower roll of upstream 177 and a clearance in the vertical direction between the upper roll of downstream 178 and the lower roll of downstream 179 may be adjusted. Steam is ejected against the aggregates 160 from the steam ejection unit 173 and the steam is sucked by the steam suction unit 174 as the aggregates 160 are compressed between the belt 110 and the first mesh conveyor belt 171. Along the parallel running segment 175, the first mesh conveyor belt 171 is shorter than the belt 110 and separated from the surface of the aggregates 160 in the vicinity of the downstream upper roll 178 so that the aggregates 160 are subjected to the suction effect by the second suction box 192 immediately below the downstream upper roll 178 in the same manner as in the fourth step 104 in the process of Fig. 5. The aggregates 160 separated from the first mesh conveyor belt 171 still lie on the belt 110 and move to the fourth step 104.

In the fourth step 104 of Fig. 6, air-permeable first web 223 is fed from above as viewed in Fig. 6 onto the upper surface of the aggregates 160 on the belt 110 facing the first mesh conveyor belt 171 as viewed in Fig. 6 to cooperate with these aggregates 160 to form the first composite web 161.

In the fifth step 105 in the process of Fig. 6, the first composite web 161 is introduced between the belt 110 and the air-permeable second endless belt 112 running in the machine direction MD. A clearance between the belt 110 and the second endless belt 112 is set substantially equal to the clearance between the belt 110 and the first mesh conveyor belt 171. Above the first composite web 161, a fourth suction box 194 is adapted to exert a vacuum suction effect on the first composite web 161 through the second endless belt 112. In the fifth step 105, the downstream lower roll 179 is used to separate the belt 110 from the aggregates 160 of the first composite web 161 under the vacuum suction effect. Exerting the vacuum suction effect on the first composite web 161, the second sheet web 224 is fed from below as viewed in Fig. 6 and the lower surface of the aggregates 160 on the first composite web 161 facing the second mesh conveyor belt 172 is covered with the second sheet web 224 to obtain the second composite web 162 comprising the aggregates 160, and the first and second sheet webs 223, 224. The second composite web 162 running in the machine direction MD is cut between each pair of the adjacent aggregates 160, 160 to obtain the individual cores 13. Thereupon, the aggregates 160 are divided into the individual compressed aggregates 20.

In such process of Fig. 6, it is possible to use an air-permeable or an air-and-liquid permeable sheet web as the first sheet web 223 and, as the second sheet web, to use not only an air-permeable sheet web but also an air-nonpermeable sheet web or an air-and-liquid-nonpermeable web sheet.

While the process exemplarily illustrated in Figs. 3 through 6 includes the step of sucking the steam streams ejected from the steam ejection unit 173 by the steam suction unit 174, the present invention can be implemented without the steam suction. For example, when the basis mass of the web to be ejected with steam streams are relatively small or the running speed of the web in the machine direction is sufficiently low so that the ejected steam streams may easily permeate the web, the steam suction unit 174 may sometimes be eliminated. The thin compressed aggregate obtained by the process according to the present invention exemplarily illustrated in Figs. 3 through 6 and the core 13 containing such compressed aggregate 20 may be used as a thin compressed aggregate or core in the bodily-fluid absorbent disposable article such as the disposable diaper 1 exemplarily illustrated in Fig. 1 and other articles such as a menstruation napkin or a urine absorbent pad. Furthermore, while the present invention has been described above on the basis of the processes exemplarily illustrated in Fig. 3 through Fig. 6 adapted to produce the thin compressed aggregates 20 in continuous fashion from the aggregate 160, the present invention may be targeted at the elemental aggregate comprising an elemental aggregate 160 or such elemental aggregate 160 wrapped with the air-permeable sheet. In this case, a pair of air-permeable supporting means comprising the first and second mesh conveyor belts 171, 172 may be replaced by a pair of air-permeable supporting means adapted to move closer to and apart from each other in the thickness direction to compress the elemental aggregate under steam ejections and thereby the thin aggregate 160 or compressed aggregate 20 may be obtained.

### (Examples 1 to 3)

In the first step through the third step illustrated in Fig. 3, fluff pulp fibers were used with a basis mass of 240g/m² as the hydrophilic fibers, SA60S available from Sumitomo Seika Chemicals Company Limited including spherical particles and aggregates of spherical particles were used with a basis mass of 240g/m² as the superabsorbent polymer particles, a through-air nonwoven fabric made of core-in-sheath type conjugate fiber having a basis mass of 25g/m² comprising polypropylene core and polypropylene sheath, and having a fineness of 2 dtex and a fiber length of 51mm was used as the first sheet web and tissue paper having a basis mass of 18g/m² was used as the second sheet web to obtain the first composite web including the aggregate of water-absorbent material having a thickness of 0.18mm and dimensions in the machine direction and the cross direction of 300mm and 200mm, respectively. The first composite web was transported at a velocity of 5m/min to the fourth step and guided into the clearance between a pair of flexible mesh conveyor belts adjusted as will be indicated later. Plain woven mesh belt of 30 meshes made of polyphenylene sulfide resin was used as the respective mesh conveyor belts. High pressure steam streams of 0. 7 MPa was ejected from nozzles of the steam ejection unit, each having a diameter of 0.5mm, arranged at a pitch of 2mm in the cross direction to the first composite web to obtain the second composite web. After being naturally dried, the second composite web was cut to obtain the individual cores according to Examples 1 through 3 each including the compressed aggregate of water-absorbent materials sandwiched between the through-air nonwoven fabric and a tissue paper. The respective clearances between the pair of mesh conveyor belts in Examples 1 through 3 were adjusted to values as indicated below.

Clearances between the pair of mesh conveyor belts:
(1) 1.6mm: Example 1
(2) 1.2mm: Example 2
(3) 0.8mm: Example 3

### (Comparative Examples 1 to 3)

In the process of the prior art illustrated in Fig. 18, the same first composite web as that used in Examples 1 through 3 in composition and in configuration was obtained. At the room temperature of 20°C, this first composite web was transported at a velocity of 5m/min in the machine direction and introduced into a nip of a compression roll pair to obtain the second composite web. Compression rolls each having a diameter of 300mm were used and compressive pressure was adjusted so that a linear pressure of 1.5kN/cm may be exerted on the aggregate of water-absorbent material fully across its width during compression of the second composite web. The respective clearances between the pair of compression rolls were adjusted to values as indicated below as (1) through (3) . The cores having been obtained with the respective clearances and having been left for 24 hours or longer were evaluated in the same way as the cores according to Comparative Examples 1 through 3.

Clearances between the pair of compression rolls:
(1) 0.6mm: Comparative Example 1
(2) 0.5mm: Comparative Example 2
(3) 0.35mm: Comparative Example 3

Not only in the cores according to Examples 1 through 3 but also in the cores according to the Comparative Examples 1 through 3, a through-air nonwoven fabric facilitating the water-absorbent materials to be peeled off from the aggregate was used as the upper sheet and thereby a potential influence of such peeling off upon the surface condition of the aggregate was negligibly alleviated during observation of the surface condition of the aggregate.

### (Evaluation of Core)

For the Examples and Comparative Examples, (1) basis mass, (2) thickness, (3) thickness recovery rate after compression, (4) absorption time, (5) absorption time under load, (6) surface smoothness, (7) airflow resistance and (8) surface condition were observed and evaluated in the manner as will be described.

### (1) Basis Mass

a. Each of the cores was cut into a size of 100mm x 100mm and was weighed. Basis mass of the first sheet web and the second sheet web was subtracted from the value corresponding to the mass of the core sample piece obtained in the manner as mentioned above and multiplied by 100 to obtain a basis mass (g/m²) of the water-absorbent material aggregate.
b. Result of measurement was recorded in TABLE 1.

### (2) Thickness

a. Each of the cores was cut into a size of 100mm x 100mm and a thickness thereof under a load of 3gf/cm² was measured using a dial gauge. Thickness of the first sheet web and the second sheet web was subtracted from the thickness of the core sample piece to obtain a thickness of the water-absorbent material aggregate.
b. A specific volume (cc/g) was calculated from (1) basis mass and (2) thickness.
c. The thickness and the specific volume obtained in this manner were recorded in TABLE 1.

### (3) Thickness Recovery Rate after Compression

a. On the cores according to the Examples and Comparative Examples, respectively, a ratio of the thickness having been measured as described in (2) versus a clearance of the mesh conveyor belt pair was obtained as a thickness recovery rate after compression r and the calculation result was recorded in TABLE 1.
b. It was observed that the thickness recovery rates r of the respective cores according to the Examples are sufficiently low to be compressed to a desired thickness without adjusting the clearance between the mesh conveyor belt pair to relatively small values required in the cores according to the Comparative Examples to be compressed to the corresponding thickness.

### (4) Absorption Time

a. After the first sheet web had been peeled off from the water-absorbent material aggregate forming the core, the core was cut into a size of 150mm x 150mm to obtain sample pieces.
b. The sample piece was placed on a horizontal plane so that the second sheet web defines the lower surface and a tip of an auto-burette was set 20mm above an upper surface of the sample piece at its central region.
c. 10cc of artificial urine was dropped from the tip of the auto-burette at a rate of 120cc/min.
d. Time elapsing from starting to drop the artificial urine to a moment at which the upper surface whitens due to absorption of the artificial urine by the sample piece was measured as the absorption time (sec). A short absorption time means that the absorption rate is high.
e. A range of the artificial urine spreading on the upper surface of the sample piece in the machine direction and the cross direction was also measured.
f. The result of the measurement was recorded in TABLE 1.
g. The artificial urine was prepared by mixing or dissolving ingredients as mentioned below in 10 liter of ion-exchanged water:

| | |
|---|---|
| Urea: | 200g |
| Sodium chloride: | 80g |
| Magnesium sulfate: | 8g |
| Calcium chloride: | 3g |
| Pigment Blue No. 1: | 1g |

h. As will be apparent from TABLE 1, on the assumption that there is no substantial difference in the sample piece thickness between the Examples and the Comparative Examples, it was observed that the sample pieces according to the Examples generally exhibit absorption rates higher than those of the sample pieces according to the Comparative Examples. It was also observed that the sample pieces according to the Examples generally exhibit absorption rates higher than those of the sample pieces according to the Comparative Examples even when the sample pieces according to Examples have thicknesses less than that of the sample pieces according to the Comparative

### Examples.

### (5) Absorption Time under Load

a. The sample pieces, same as those used for measurement of the absorption time (4), were prepared.
b. A liquid-pervious nonwoven fabric piece having a size of 40mm x 40mm (Bemliese PS140 manufactured by Asahi Kasei Corporation) was placed on the upper surface of the sample piece at a central region thereof.
c. The sample piece and the measuring device were set as illustrated in Fig. 7 and the tip of the auto-burette was positioned, within a transparent cylinder as illustrated, 20mm above the upper surface of the sample pieces.
d. 20cc of the artificial urine was dropped onto the sample piece at a rate of 120cc/min.
e. Observing the interior of the cylinder, time elapsing from the moment at which dropping of the artificial urine had been started to the moment at which the artificial urine had been completely absorbed by the sample piece was measured to obtain the absorption time (sec) under load. The shorter the absorption time under load, the higher the absorption rate under load is.
f. A range of the artificial urine spreading on the upper surface of the sample piece in the machine direction and the cross direction was also measured.
g. Results of the measurement were recorded in TABLE 1.

### (6) Surface Smoothness

a. The core was cut into a size of 100mm x 100mm and the through-air nonwoven fabric used as the first sheet web in the process for making the core was peeled off from the aggregate of water-absorbent material to obtain a sample piece for measurement. The sample piece was placed on a horizontal reference surface with the lower surface of the core as its downside and values of height from the reference surface to respective regions on the upper surface of the sample piece were measured to obtain variation in the height corresponding to irregularity of the upper surface. Based on a degree of the variation, quality of the surface smoothness in the aggregate of water-absorbent material was evaluated.
b. As measuring means, High-Accuracy Geometry Measuring System (inclusive of High-Accuracy Stage: KS-1100) and High-Speed and High-Accuracy CCD-Laser Displacement Gauge inclusive of Controller: LK-G3000V Set and Sensor Head: LK-G30) manufactured by Keyence Corporation were used.
c. Stage conditions were set as following:

| | |
|---|---|
| Range of measurement: | 40000µm x 40000µm |
| Measuring pitch: | 20µm |
| Running speed: | 7500µm/sec |

d. Conditions of the measuring head (LK-G3000) were set as following:

| | |
|---|---|
| Measurement mode: | Object to be measured |
| Installation mode: | Diffuse reflection |
| Filtering: | four (4) times in average |
| Sampling period: | 200µs |

e. The data obtained on the basis of the sample piece (a) was processed by using a configuration analysis software (KS-H1A). The data having been processed was transferred to Excel, spreadsheet software of Microsoft, by extracting Z-coordinates each associated with 16 spots on the X- and Y-coordinates, respectively. Using this software, a contour graph was created on the basis of X-, Y- and Z-coordinates. Furthermore, histogram processing of all Z-coordinates was carried out utilizing add-in features of this software.
f. These measurements and processing were carried out on the respective cores according to Example 3 and Comparative Example 3 and the results thereof were illustrated in Figs. 8, 9 and 10. Figs. 8 and 9 visualize variation of surface irregularity of the sample piece wherein the ordinate indicates the height from the reference surface to the upper surface of the sample piece. It should be noted here that all the measurement points each extracted for sixteen (16) spots in X-and Y-coordinates, respectively, would complicate the graph and make it difficult to observe the variation in irregularity. To solve this problem, one measurement point was further extracted from four (4) already extracted measurement points, thereby Z-coordinate values each for 1280µm were extracted and values thereof were processed by Excel. The data processing method employed by the present invention allows the measurement result to be illustrated in the form of a polygonal line graph wherein, for example, X-coordinates are fixed and Y-coordinates are left variable to indicate values of Z-coordinates varying in association with the respective Y-coordinates. While such a polygonal line graph can be separated by color depending on values of the fixed X-coordinates, the polygonal line graph of Figs. 8 and 9 are illustrated simply by black lines. In Fig. 10, the height from the reference surface to the upper surface is indicated by abscissa axis and the frequency (the number of times) at which the height was detected is indicated by axis of ordinate. Degree of irregularity on the upper surface was evaluated to be insignificant in the sample pieces according to the Examples in comparison with that observed in the sample pieces according to the Comparative Examples. In other words, the sample pieces according to the Examples demonstrated a surface smoothness higher than that of the sample pieces according to the Comparative Examples.

### (7) Airflow Resistance

a. Each of the cores was cut in a circular shape having a diameter of 88mm, then the first sheet web and the second sheet web were peeled off from the aggregate of water-absorbent material to obtain a sample piece.
b. AIR PERMEABILITY TESTER: KES-F8-APL (manufactured by KATO TECH CO., LTD.) was set to a standard airflow velocity of 2cm/sec to measure the airflow resistance value of the sample piece in its dry state.
c. The sample piece was left as it is for one (1) minute after the sample piece had absorbed 20cc of the artificial urine and the airflow resistance value was measured on this sample piece in the same manner as described in paragraph b to obtain the airflow resistance value in its wet state.
d. Airflow resistance values were divided by basis masses of the respective sample pieces to obtain the airflow resistance indices for comparison.
e. Results of measurements obtained for the Examples and Comparative Examples were recorded in TABLE 2.

### (8) Surface Condition

a. The core was cut into a size of 100mm x 100mm and the first sheet web formed by through-air nonwoven fabric was peeled off from the aggregate of water-absorbent material to obtain a sample piece.
b. Using Real Surface View Microscope VE-7800 (manufactured by Keyence Corporation), the upper surface of the sample piece having the first sheet web peeled off therefrom was photographed at magnifications of x 50 and x 100 and the surface condition of the sample piece was observed on these photos.
c. As objects to be observed, the sample pieces according to EXAMPLE 3 and Comparative Example 3 were selected. Figs. 11 and 12 are x 50 and x 100 photos of the core according to EXAMPLE 3 and Figs. 13 and 14 are x 50 and x 100 photos of the core according to Comparative Example 3.
d. Figs. 11 through 14 demonstrate that the superabsorbent polymer particles partially collapse in the core according to Comparative Example 3. More specifically, the particles are separated one from another and some of these particles separated one from another exhibit partial collapse on the spherical surfaces thereof. In addition, the superabsorbent polymer particles and the pulp fibers are in the same vicinity as one another and the pulp fibers themselves also are in the same vicinity as one another in the Comparative Example 3. In the core according to Example 3, no collapse can be recognized on the superabsorbent polymer particles and appropriate clearances are kept between the particles and the pulp fibers and between each pair of the adjacent pulp fibers, respectively.

**(TABLE 1)**

| Evaluation Items | Example1 | Example2 | Example3 | Comparative Example1 | Comparative Example2 | Comparative Example3 |
|---|---|---|---|---|---|---|
| (1)Basis mass(g/m²) | 525.5 | 523.1 | 533.9 | 510.7 | 546.6 | 508.2 |
| (2)Thickness(mm) | 3.0 | 2.6 | 2.1 | 3.2 | 2.5 | 2.0 |
| Specific volume(cc/g) | 5.7 | 4.9 | 3.9 | 6.4 | 4.5 | 3.9 |
| (3)thickness recovery rate after compression | 1.9 | 2.3 | 2.6 | 5.7 | 5.6 | 6.3 |
| (4)Absorption time | | | | | | |
| Absorption time(sec) | 31.6 | 29.8 | 23.6 | 43.2 | 31.9 | 27.9 |
| Dispersion range in MD direction(mm) | 73 | 82 | 82 | 74 | 92 | 90 |
| Dispersion range in CD direction(mm) | 68 | 77 | 82 | 63 | 79 | 99 |
| (5)Absorption time under load | | | | | | |
| Absorption time(sec) | 21.9 | 29.8 | 52.8 | 21.6 | 38.4 | 66.4 |
| Dispersion range in MID direction(mm) | 114 | 115 | 109 | 112 | 111 | 111 |
| Dispersion range in CD direction(mm) | 117 | 116 | 106 | 121 | 124 | 115 |

**(TABLE 2)**

| Evaluation Items(7) | Example1 | Example2 | Example3 | Comparative Example 1 | Comparative Example2 | Comparative Example3 |
|---|---|---|---|---|---|---|
| Airflow resistance(in dry state) | | | | | | |
| Airflow resistance index | 0.000287 | 0.000285 | 0.000316 | 0.000287 | 0.000359 | 0.000608 |
| Basis mass of test piece (g/m²) | 489.5 | 430.8 | 436.3 | 448.9 | 461.0 | 481.3 |
| Thickness of test piece(mm) | 3.0 | 2.5 | 1.9 | 3.5 | 2.8 | 1.9 |
| Specific volume of test piece(cc/g) | 6.2 | 5.8 | 4.4 | 7.7 | 6.0 | 4.0 |
| Airflow resistance(in wet state) | | | | | | |
| Airflow resistance index | 0.000598 | 0.000730 | 0.000710 | 0.000909 | 0.000877 | 0.001075 |
| Basis mass of test piece(g/m²) | 435.9 | 427.2 | 431.0 | 436.3 | 457.7 | 435.2 |
| Thickness of test piece(mm) | 2.8 | 2.5 | 2.3 | 3.2 | 2.7 | 2.1 |
| Specific volume of test piece(cc/g) | 6.4 | 5.7 | 5.3 | 7.4 | 5.9 | 4.8 |

Fig. 15 is a graphic diagram plotting relationships between the absorption rates and the specific volumes both recorded in Table 1. Of the cores according to the Examples and the cores according to the Comparative Examples having one and the same specific volume, the core which is most preferable in view of the highest absorption rate is the core 13 according to the Example (See Figs. 11 and 12) wherein adequate clearance is kept between each pair of the adjacent hydrophilic fibers 21 and between the hydrophilic fibers 21 and the adjacent superabsorbent polymer particles.

Fig. 16 is a graphic diagram plotting relationships between the absorption times under load and the specific volumes both recorded in TABLE 1. So far as the cores according to the Examples and the Comparative Examples, which were selected as the observation objects, are concerned, the pattern in which the absorption time under load is shortened was observed, i.e., the absorption rate under load is accelerated as the specific volume sizes up. Such pattern was common to the Examples and Comparative Examples.

Fig. 17 is a graphic diagram plotting relationships between the airflow resistance indices and the specific volumes both recorded in TABLE 2. Of the cores according to the EXAMPLES and the cores according to the Comparative Examples, the pattern was observed in which the airflow resistance indices of the cores according to the Examples are lower than the airflow resistance indices of the cores according to the Comparative Examples. Such pattern was observed on the cores in a dry state and on the cores in a wet state. The cores according to the Examples having relatively low airflow resistance indices are the cores having low vapor flow resistance values and correspondingly high air-and-liquid permeability.

### {Reference Signs List}

- 13: core
- 21: water-absorbent fiber
- 22: superabsorbent polymer particles
- 160: aggregate
- MD: machine direction
- TD: thickness direction

## Claims

1. A method for thinning an aggregate (160) of water-absorbent material including hydrophilic fibers (21) and superabsorbent polymer particles (22) in a thickness direction (TD) of the aggregate, the method comprising a step of:
ejecting steam streams at a temperature corresponding to water's boiling point or higher against the aggregate while the aggregate is compressed in the thickness direction to thin the aggregate;
wherein the aggregate is sandwiched and compressed by a pair of air-permeable supporting means (171,172) opposed to each other in the thickness direction of the aggregate while the steam streams are ejected against the aggregate in the thickness direction through any one of the pair of air-permeable supporting means.

2. The method defined by Claim 1, wherein the steam streams are any one of moist steam streams, saturated steam streams and dry steam streams.

3. The method defined by Claim 1 or 2, wherein the steam streams are high pressure steam at a steam pressure in a range of 0.1 to 2.0MPa.

4. The method defined by any preceding claim, wherein the steam streams are sucked under vacuum suction effect after the steam has been ejected against the aggregate and has passed through the aggregate.

5. The method defined by any preceding claim, wherein the steam streams in a range of 1.23kg/m² to 0.03kg/m² of surface area of the aggregate facing one of the pair of air-permeable supporting means are ejected while the pair of air-permeable supporting means run at a velocity in a range of 5 to 500m/min in one direction.

6. The method defined by any preceding claim, wherein the pair of air-permeable supporting means respectively include segments adapted to sandwich the aggregate and to run in any one of a horizontal direction, a vertical direction and a tilted direction between these horizontal and vertical directions.

7. The method defined by any preceding claim, wherein each of the pair of air-permeable supporting means is an endless belt.

8. The method defined by any preceding claim, wherein the aggregate is previously thinned by mechanically compressing the aggregate in the thickness direction and thereafter the steam streams are ejected against the aggregate kept under compression.

9. The method defined by Claim 8, wherein the aggregate is mechanically compressed using at least a pair of pressure rolls for previous thinning of the aggregate.

10. The method defined by any preceding claim, wherein at least one of a side facing one of the pair of air-permeable supporting means and a side facing the other of the pair of air-permeable supporting means of the aggregate is covered with any one of an air-permeable sheet and an air-permeable and liquid-pervious sheet and thereafter the aggregate is sandwiched between the pair of air-permeable supporting means.

11. The method defined by any preceding claim, wherein, after being subjected to ejection of the steam streams, one of a side of the aggregate facing the one of the pair of air-permeable supporting means and the other side of the aggregate facing the other of the pair of air-permeable supporting means is covered with one of an air-permeable sheet, an air-permeable and liquid-pervious sheet and an non-air-permeable sheet.

12. The method defined by any preceding claim, wherein, in the step of separating the aggregate, after having been subjected to ejection of the steam streams, from the one of the pair of air-permeable supporting means and the other of the pair of air-permeable supporting means, the aggregate is subjected to the vacuum suction effect through any one of the pair of air-permeable supporting means.

13. The method defined by any preceding claim, wherein the aggregate includes the hydrophilic fibers in a range of 98 to 10% by mass and the superabsorbent polymer particles in a range of 2 to 90% by mass.

14. The method defined by any preceding claim, wherein the hydrophilic fibers may be selected from a group comprising fluff pulp fibers, cotton fibers, rayon fibers, acetate fibers and thermoplastic synthetic fibers modified to become hydrophilic.

15. The method defined by any preceding claim, wherein the superabsorbent polymer particles may be selected from particles of polyacrylic acid, polyacrylate, starch-acrylonitrile graft copolymer, polyvinyl alcohol, polyvinyl ether, polyacrylamide, carboxymethyl cellulose or natural polysaccharide.

16. The method defined by any one of Claims 10 through 15, wherein any one of the air-permeable sheet and the air-permeable and liquid-pervious sheet is any one of a tissue paper and a nonwoven fabric.

17. A water-absorbent material aggregate made by the method defined by any one of Claims 1 through 16.

## Patentansprüche

1. Verfahren zum Verdünnen eines Aggregats (160) eines wasserabsorbierenden Materials, das hydrophile Fasern (21) und superabsorbierende Polymerteilchen (22) in Dickenrichtung (TD) des Aggregats beinhaltet, wobei das Verfahren den folgenden Schritt umfasst:
Ausstoßen von Dampfströmen bei einer Temperatur, die mit dem Siedepunkt des Wassers, oder höher, am Aggregat korrespondiert, während das Aggregat in Dickenrichtung zusammengedrückt wird, um das Aggregat zu verdünnen,
wobei das Aggregat zwischen einem Paar luftdurchlässiger Stützmittel (171, 172) eingelegt und zusammengedrückt wird, die gegenüber voneinander in Dickenrichtung des Aggregats liegen, während die Dampfströme gegen das Aggregat in Dickenrichtung durch eines der Paare von luftdurchlässigen Stützmitteln ausgestoßen werden.

2. Verfahren nach Anspruch 1, wobei die Dampfströme eine der feuchten Dampfströme, der gesättigten Dampfströme und der trockenen Dampfströme aind.

3. Verfahren nach Anspruch 1 oder 2, wobei die Dampfströme ein Hochdruckdampf mit einem Dampfdruck im Bereich von 0,1 bis 2,0 Mpa sind.

4. Verfahren nach einem vorangehenden Anspruch, wobei die Dampfströme unter Saugwirkung des Vakuums angesaugt werden, nachdem der Strom gegen das Aggregat ausgestoßen wurde und durch das Aggregat gegangen ist.

5. Verfahren nach einem der vorangehenden Ansprüche, wobei die Dampfströme in einem Bereich von 1,23 kg/m² bis 0,03 kg/m² des Oberflächenbereichs des Aggregats einem der Paare von luftdurchlässigen Stützmitteln gegenübersteht, während das Paar von luftdurchlässigen Stützmitteln mit einer Geschwindigkeit in einem Bereich von 5 bis 500 m/min in einer Richtung läuft.

6. Verfahren nach einem vorangehenden Anspruch, wobei das Paar von luftdurchlässigen Stützmitteln jeweils Segmente beinhaltet, die adaptiert wurden, um das Aggregat dazwischen einzulegen, und in einer waagrechten Richtung, einer senkrechten Richtung und einer schrägen Richtung zwischen diesen waagrechten und senkrechten Richtungen zu laufen.

7. Verfahren nach einem vorangehenden Anspruch, wobei jedes der Paare von luftdurchlässigen Stützmitteln ein Endlosband ist.

8. Verfahren nach einem vorangehenden Anspruch, wobei das Aggregat vorher durch mechanisches Zusammendrücken des Aggregats in Dickenrichtung verdünnt wird, und danach die Dampfströme gegen das Aggregat, das unter Druck gehalten wird, ausgestoßen wird.

9. Verfahren nach Anspruch 8, wobei das Aggregat mechanisch mit Hilfe von wenigstens einem Paar von Druckwalzen für das vorhergehende Verdünnen des Aggregats zusammengedrückt wird.

10. Verfahren nach einem vorangehenden Anspruch, wobei wenigstens eine Seite, die einem Paar von luftdurchlässigen Stützmitteln gegenüberliegt, und eine Seite, die dem anderen Paar von luftdurchlässigen Stützmitteln des Aggregats mit einer der luftdurchlässigen Schicht und einer luftdurchlässigen und flüssigkeitsdurchlässigen Schicht abgedeckt wurde, und anschließend das Aggregat zwischen dem Paar der luftdurchlässigen Stützmittel gelegt ist.

11. Verfahren nach einem vorangehenden Anspruch, wobei, nachdem es einem Auswurf der Dampfströme ausgesetzt wurde, eine der Seiten des Aggregats steht, das einem Paar von luftdurchlässigen Stützmitteln gegenübersteht, und die andere Seite des Aggregats, das dem anderen Paar von luftdurchlässigen Stützmitteln gegenübersteht, mit einer luftdurchlässigen Schicht, einer luftdurchlässigen nd flüssigkeitsdurchlässigen Schicht und einer luftundurchlässigen Schicht abgedeckt ist.

12. Verfahren nach einem vorangehenden Anspruch, wobei während des Schritts des Trennens des Aggregats, nachdem es einem Auswurf der Dampfströme ausgesetzt wurde, von einem Paar von luftdurchlässigen Stützmitteln und dem anderen Paar von luftdurchlässigen Stützmitteln, wobei das Aggregat einer Saugwirkung des Vakuums durch irgendein Paar von luftdurchlässigen Stützmitteln ausgesetzt ist.

13. Verfahren nach einem vorangehenden Anspruch, wobei das Aggregat die hydrophilen Fasern in einem Bereich von 98 bis 10 % nach Masse und die superabsorbierenden Polymerteilchen in einem Bereich von 2 bis 90 % nach Masse beinhaltet.

14. Verfahren nach einem vorangehenden Anspruch, wobei die hydrophilen Fasern von einer Gruppe, die Flaumzellstofffasern, Baumwollfasern, Viskosefasern, Acetatfasern und thermoplastische synthetische Fasern enthält, so modifiziert sind, dass sie hydrophil werden.

15. Verfahren nach einem vorangehenden Anspruch, wobei die superabsorbierenden Polymerteile von Teilen von polyacryler Säure, Polyacrylat, stärkenakrylnitrilem Pfropfcopolymer, Polyvinylalkohol, Polyvinylether, Polyacrylamid, Carbomethyl-Zellstoff oder natrürlichem Polysaccharid gewählt werden.

16. Verfahren nach einem der Ansprüche 10 bis 15, wobei irgendeine der luftdurchlässigen Schichten und die luftdurchlässige und flüssigkeitsdurchlässige Schicht eine von einem Seidenpapier und einem Vliesstoff ist.

17. Wasserabsorbierendes Materialaggregat, das mit dem Verfahren, das in einem der Ansprüche 1 bis 16 definiert ist, gemacht wurde.

## Revendications

1. Procédé d'amincissement d'un agrégé (160) de matière absorbant l'eau comprenant des fibres hydrophiles (21) et des particules de polymère superabsorbant (22) dans une direction d'épaisseur (TD) de l'agrégé, le procédé comprenant les étapes consistant à :
éjecter des jets de vapeur à une température correspondant au point d'ébullition de l'eau ou plus élevée contre l'agrégé tandis que l'agrégé est comprimé dans la direction de l'épaisseur afin d'amincir l'agrégé;
dans lequel l'agrégé est en sandwich entre et comprimé par une paire de moyens de support perméables à l'air (171, 172) opposés l'un à l'autre dans la direction de l'épaisseur de l'agrégé pendant que les jets de vapeur sont éjectés contre l'agrégé dans la direction de l'épaisseur à travers l'un quelconque de la paire de moyens de support perméables à l'air.

2. Procédé selon la revendication 1, dans lequel les jets de vapeur sont l'un quelconque d'entre des jets de vapeur humide, des jets de vapeur saturée et des jets de vapeur sèche.

3. Procédé selon la revendication 1 ou 2, dans lequel les jets de vapeur sont de la vapeur à haute pression à une pression de vapeur dans une plage de 0,1 à 2,0 MPa.

4. Procédé selon l'une quelconque des revendications précédentes, dans lequel les jets de vapeur sont aspirés sous l'effet d'aspiration à vide après que la vapeur a été éjectée contre l'agrégé et est passée à travers l'agrégé.

5. Procédé selon l'une quelconque des revendications précédentes, dans lequel les jets de vapeur dans une plage de 1,23 kg/m² à 0,03 kg/m² d'aire de surface de l'agrégé faisant face à l'un de la paire de moyens de support perméables à l'air, sont éjectés tandis que la paire de moyens de support perméables à l'air se déplace à une vitesse dans une plage de 5 à 500 m/min dans une direction.

6. Procédé selon l'une quelconque des revendications précédentes, dans lequel la paire de moyens de support perméables à l'air comprend respectivement des segments adaptés pour mettre l'agrégé en sandwich et se déplacer dans l'une quelconque d'entre une direction horizontale, une direction verticale et une direction inclinée entre ces directions horizontale et verticale.

7. Procédé selon l'une quelconque des revendications précédentes, dans lequel chacun de la paire de moyens de support perméables à l'air est une courroie sans fin.

8. Procédé selon l'une quelconque des revendications précédentes, dans lequel l'agrégé est précédemment aminci mécaniquement en comprimant l'agrégé dans la direction de l'épaisseur et ensuite les jets de vapeur sont éjectés contre l'agrégé maintenu en compression.

9. Procédé selon la revendication 8, dans lequel l'agrégé est comprimé mécaniquement en utilisant au moins une paire de rouleaux de pression pour l'amincissement précédent de l'agrégé.

10. Procédé selon l'une quelconque des revendications précédentes, dans lequel au moins l'un d'un côté faisant face à l'un de la paire de moyens de support perméables à l'air et d'un côté faisant face à l'autre de la paire de moyens de support perméables à l'air de l'agrégé est couvert avec l'une quelconque d'entre une feuille perméable à l'air et une feuille perméable à l'air et perméable au liquide et l'agrégé est ensuite en sandwich entre la paire de moyens de support perméables à l'air.

11. Procédé selon l'une quelconque des revendications précédentes, dans lequel, après avoir été soumis à l'éjection des jets de vapeur, l'un d'entre un coté de l'agrégé faisant face à l'un de la paire de moyens de support perméables à l'air et l'autre côté de l'agrégé faisant face à l'autre de la paire de moyens de support perméables à l'air est couvert avec l'une d'entre une feuille perméable à l'air, une feuille perméable à l'air et perméable au liquide et une feuille non perméable à l'air.

12. Procédé selon l'une quelconque des revendications précédentes, dans lequel, à l'étape de séparation de l'agrégé de l'un de la paire de moyens de support perméables à l'air et de l'autre de la paire de moyens de support perméables à l'air, après avoir été soumis à l'éjection des jets de vapeur, l'agrégé est soumis à l'effet d'aspiration à vide à travers l'un quelconque de la paire de moyens de support perméables à l'air.

13. Procédé selon l'une quelconque des revendications précédentes, dans lequel l'agrégé comprend les fibres hydrophiles dans une plage de 98 à 10 % en masse et les particules de polymère superabsorbant dans une plage de 2 à 90 % en masse.

14. Procédé selon l'une quelconque des revendications précédentes, dans lequel les fibres hydrophiles peuvent être sélectionnées parmi le groupe comprenant des fibres de pulpe pelucheuses, des fibres de coton, des fibres de rayonne, des fibres d'acétate et des fibres synthétiques thermoplastiques modifiées pour devenir hydrophiles.

15. Procédé selon l'une quelconque des revendications précédentes, dans lequel les particules de polymère superabsorbant peuvent être sélectionnées parmi des particules d'acide polyacrylique, de polyacrylate, de copolymère greffé d'amidon-acrylonitrile, d'alcool polyvinylique, d'éther de polyvinyle, de polyacrylamide, de carboxyméthylcellulose ou de polysaccharide naturel.

16. Procédé selon l'une quelconque des revendications 10 à 15, dans lequel l'une quelconque d'entre la feuille perméable à l'air et la feuille perméable à l'air et perméable au liquide est l'un quelconque d'entre un papier soie et un tissu non tissé.

17. Agrégé de matière absorbant l'eau fabriqué par le procédé selon l'une quelconque des revendications 1 à 16.
